# FASCICULE DE BREVET EUROPEEN

(11) **EP 2 804 647 B1**
(45) Date de publication et mention de la délivrance du brevet: **28.12.2016**
(21) Numéro de dépôt: 13704622.3
(22) Date de dépôt: 18.01.2013
(51) Int. Cl.: A61M 11/00, A61M 15/00, B05B 17/06

(54) **DISPOSITIF DE NEBULISATION POUR AEROSOLS MEDICAUX**
VERNEBLERVORRICHTUNG FÜR MEDIZINISCHE AEROSOLE
NEBULIZER DEVICE FOR MEDICAL AEROSOLS

(30) Priorité: 20.01.2012 FR 1250569
(43) Date de publication de la demande: 26.11.2014
(73) Titulaire: LA DIFFUSION TECHNIQUE FRANCAISE, 42000 Saint Etienne (FR)
(72) Inventeur: VECELLIO-NONE, Laurent, 31170 Chambray Les Tours (FR); CHANTREL, Gilles, 42100 Saint-Etienne (FR); MASSARDIER, Michel, 42000 Saint-Etienne (FR)
(74) Mandataire: Cabinet Laurent & Charras
(86) Numéro de dépôt international: PCT/FR2013/050114
(87) Numéro de publication internationale: WO 2013/107992

(56) Documents cités:
- EP-A1- 1 674 122
- EP-A1- 1 743 671
- EP-A1- 2 457 609
- EP-B1- 1 927 373
- WO-A1-01/34232
- WO-A1-99/47196
- WO-A1-2009/013501
- WO-A2-2010/008424
- DE-A1- 10 102 846
- DE-B3-102005 006 374
- DE-U1- 20 115 203
- US-A1- 2005 056 274

## Description

L'invention se rattache au secteur technique des systèmes de génération d'aérosols médicaux.

Les systèmes de génération d'aérosols médicaux ont pour fonction de transformer, un liquide ou une poudre, médicamenteux sous forme d'aérosol pour être administré dans les voies aériennes respiratoires.

Divers systèmes de génération d'aérosols médicaux existent sur le marché avec des sous formes d'appareils à commandes pneumatiques, ultra soniques, à tamis, notamment, ainsi que des flacons pressurisés avec valve doseuse. Les appareils de nébulisation sont utilisés pour délivrer des quantités importantes de médicaments variés directement dans les voies aériennes des patients. Ils procurent ainsi une action thérapeutique rapide et sélective au niveau même du site pathologique. La nébulisation peut être définie comme la transformation d'un liquide médicamenteux en aérosol médicamenteux. On emploie le terme « nébuliser » comme synonyme de « transformer un liquide en aérosol ». L'appareil de nébulisation comporte un réservoir dans lequel est introduit le liquide à nébuliser, une chambre de nébulisation, lieu de production de l'aérosol, et une source d'énergie - pneumatique ou piézo-électrique - utilisée pour créer l'aérosol.

Trois principes de nébulisation sont actuellement utilisés pour produire un aérosol médicamenteux : la nébulisation pneumatique, la nébulisation ultrasonique et plus récemment la nébulisation à tamis.

Les systèmes de nébulisation à tamis sont des nébuliseurs de nouvelle génération. Ces systèmes sont silencieux, et peuvent être portables et de petite taille. Ils sont composés d'un boîtier d'alimentation électronique raccordé au nébuliseur. Le boîtier d'alimentation électronique peut fonctionner sur secteur ou sur piles. Le générateur d'aérosol est composé d'un tamis percé d'une grande quantité de trous microscopiques (de 1000 à 6000 trous selon les modèles). Ces trous, d'un diamètre de 2µm à 10 µm sont obtenus par électrophorèse ou perçage laser du tamis. Le passage du liquide médicamenteux à travers le tamis transforme le liquide en gouttelettes d'aérosol de taille équivalente à la taille des trous du tamis.

Actuellement, il existe deux grands types de générateurs d'aérosol à tamis, celui à tamis fixe et celui à tamis vibrant.

Le générateur d'aérosol à tamis fixe utilise un piézo-électrique en contact avec le liquide médicamenteux. La vibration du piézo-électrique (entre 50KHz et 500KHz) est transmise au liquide forçant son passage à travers les trous du tamis pour détacher des gouttelettes respirables vers l'extérieur du tamis. L'aérosol produit est ensuite mobilisé par le patient hors du nébuliseur.

Le générateur d'aérosol à tamis vibrant utilise un piézo-électrique entourant le tamis pour le mettre en vibration (entre 50KHz et 500KHz). La solution médicamenteuse alors en contact avec le tamis va être aspirée par les trous coniques lorsqu'il se déforme vers le réservoir de solution. Lorsque le tamis se déformera vers l'extérieur, la solution alors située au bord du tamis va se détacher pour former des particules respirables. L'aérosol est ensuite mobilisé par le patient hors du nébuliseur. Avec un tel système, les pertes de médicaments dans le réservoir du nébuliseur sont négligeables et le débit d'aérosol produit est comparable à celui des nébuliseurs ultrasoniques (environ 0.5ml/min).

Les systèmes de génération d'aérosol médicaux sont composés d'un générateur d'aérosol à proprement dit et d'une interface entre le générateur d'aérosol et le patient. Le générateur d'aérosol est la source de génération de l'aérosol. L'interface patient permet le transport de l'aérosol, depuis le générateur vers le patient (exemples : masque, embout buccal, embout narinaire, circuit de ventilateur mécanique, sonde d'intubation, sonde trachéale...). Certains appareils générateurs d'aérosol médicamenteux qui produisent un aérosol en continu peuvent générer une perte de médicament dans l'atmosphère durant la phase expiratoire du patient. En effet, lorsque le patient expire dans l'appareil générateur, ou n'inspire pas dans l'appareil générateur, l'aérosol peut s'échapper librement de l'appareil générateur. C'est le cas par exemple des nébuliseurs Aeroneb Go ou Micro air. L'aérosol est produit par le générateur d'aérosol dans un volume possédant deux ouvertures : la première ouverture est destinée à être connectée au patient et la deuxième ouverture est libre et permet le passage d'air inspiré et expiré par le patient. Ainsi, lors de la phase inspiratoire du patient, l'air inspiré par le patient traverse la deuxième ouverture et transporte l'aérosol vers le patient. Lors de la phase expiratoire buccale du patient, l'air expiré par le patient transporte avec lui l'aérosol produit par le générateur d'aérosol et est expulsé dans l'air ambiant à travers la deuxième ouverture. Ainsi le système de nébulisation génère de fortes pertes d'aérosols durant la phase expiratoire du patient.

Afin de limiter les pertes d'aérosol durant la phase expiratoire du patient, une zone intermédiaire peut être ajoutée entre la zone de génération de l'aérosol et l'interface. Cette zone intermédiaire est appelée zone de stockage. L'ensemble zone de stockage et interface, forme alors la chambre d'inhalation ayant pour fonction de stocker l'aérosol durant les phases d'expiration du patient et d'administrer l'aérosol stocké lors des phases d'inspiration du patient. Différents moyens techniques permettent d'assurer le fonctionnement de la chambre d'inhalation selon les phases respiratoires du patient.

La demande de la société Pari (WO/0134232A1) décrit un système de nébulisation à tamis utilisant une chambre d'inhalation possédant un double système de valve tel que lors de la phase expiratoire, l'air expiré par le patient est expulsé a travers une première valve expiratoire sans traverser la zone de stockage et tel que lors de la phase inspiratoire, l'air qui pénètre dans la zone de stockage à travers la valve inspiratoire transporte l'aérosol vers le patient. La valve inspiratoire étant fermée lors de la phase expiratoire et ouverte lors de la phase inspiratoire ; la valve expiratoire étant ouverte durant la phase expiratoire et fermée durant la phase inspiratoire.

Une autre demande de la société Pari (US2005/056274A1) décrit un dispositif comportant une cavité dont la fonction est de transporter l'aérosol produit. Cette cavité peut être assimilée à un unique conduit de transport de l'aérosol tel qu'un embout buccal. Cette cavité n'a pas la fonction d'une chambre d'inhalation, au sens de la présente invention.

Une autre demande de la société Pari (DE20115203U1) décrit un dispositif comportant une ouverture munie d'une valve expiratoire unidirectionnelle au niveau de l'embout buccal.

La demande du laboratoire Novartis (WO2010008424) décrit une chambre utilisant une unique valve placée sur la zone de stockage et une résistance à l'écoulement de l'air ci après dénommée résistance d'air placée sur l'interface (filtre par exemple). Dans ces conditions, l'air expiré par le patient est expulsé par le filtre sans traverser la zone de stockage, et l'air inspiré traverse principalement la zone de stockage pour transporter l'aérosol vers le patient par l'intermédiaire de l'interface. Ce fonctionnement de la chambre d'inhalation ne peut être assuré que par l'utilisation d'une valve à faible résistance ayant pour conséquence un dispositif avec une forte résistance à l'expiration et une faible résistance à l'inspiration.

La demande de brevet au nom de la Diffusion Technique Française (EP 1 743 671) décrit une chambre d'inhalation n'utilisant pas de valves mais telle que les circuits d'air expirés et inspirés soient différents. Dans ces conditions, l'air expiré par le patient est expulsé de la chambre sans traverser la zone de stockage et l'air inspiré traverse la zone de stockage pour transporter l'aérosol vers le patient par l'intermédiaire de l'interface. Le fonctionnement de cette chambre impose la contrainte de non résistance des ouvertures afin de ne pas créer de surpression dans la chambre ayant pour conséquence un dispositif avec une faible résistance à l'expiration et à l'inspiration.

Par ailleurs, il est admis que le débit inspiratoire du patient est un paramètre important. Un faible débit inspiratoire permet d'améliorer le dépôt pulmonaire en comparaison d'un fort débit inspiratoire. En effet, les particules transportées avec un plus faible débit inspiratoire auront une vitesse moins importante limitant ainsi le phénomène physique d'impaction au niveau oropharyngé. Ces particules avec une faible vitesse pourront ainsi pénétrer dans les poumons sans dépôt dans les voies aériennes supérieures. Par exemple, l'utilisation d'une résistance à l'air durant la phase inspiratoire du patient permet de limiter le débit d'air inspiré afin d'améliorer le traitement. Cette résistance est physiquement réalisée par l'utilisation d'une ouverture à faible diamètre créant une résistance à l'écoulement de l'air inspiré par le patient. Cette ouverture est placée sur la zone de stockage en amont du générateur d'aérosol afin de limiter la vitesse des particules lors de la pénétration de celle ci dans le patient. Par comparaison, une ouverture de faible diamètre placée en aval du générateur d'aérosol aurait pour conséquence une augmentation de la vitesse des particules administrées au patient. Selon les précédentes descriptions des principes de fonctionnement des systèmes de nébulisation à tamis utilisant des chambres d'inhalation, seule l'utilisation d'un système de double valve onéreux (WO/0134232A1) permet une limitation du débit inspiratoire du patient par l'utilisation d'une résistance placée au niveau de la valve inspiratoire, en amont du générateur d'aérosol. Les principes de fonctionnement des chambres d'inhalation utilisant une valve ou aucune valve ne permettent actuellement pas de limiter le débit inspiratoire par l'utilisation d'une résistance à l'air placée en amont du générateur d'aérosol.

Le Déposant est l'un des leaders dans la fabrication et la commercialisation de ce type d'appareils.

Face à cette situation, le Demandeur s'est alors orienté sur une conception différente de ce type d'appareils qui permet la réduction des pertes d'aérosol par stockage de l'aérosol durant la phase expiratoire du patient tout en assurant une limitation du débit inspiratoire du patient et en réduisant les coûts d'industrialisation.

La solution apportée va à l'encontre des solutions connues en étant en opposition complète par rapport à leurs mises en oeuvre et fonctionnements d'appareils nébuliseurs correspondants.

Le dispositif de nébulisation de la présente invention est tel que défini dans la revendication 1.

Selon l'invention, la chambre d'inhalation est conçue de telle sorte que la deuxième ouverture a une résistance moyenne supérieure à la résistance moyenne de la dite troisième ouverture et de la valve expiratoire pour les différentes valeurs de débits expiratoires, permettant ainsi de dissocier les circuits d'air inspirés et expirés dans le dispositif.

La solution retenue est donc à l'opposé du fonctionnement des chambres d'inhalation rappelées précédemment.

Ainsi, par la solution de l'invention, le débit expiratoire du patient pouvant être modélisé par une fonction sinusoïdale en fonction du temps, les premiers mL d'air expiré par le patient correspondent à des valeurs de débits de l'ordre de quelques litres/min. Ces valeurs de faibles débits sont en accord avec la sensibilité des valves mécaniques à faible résistance disponibles sur le marché (résistance inférieure à 0.07 cm H20 min/L). Ainsi, l'utilisation d'une valve de faible résistance permet d'assurer le fonctionnement du système dès le début de l'expiration du patient et de maintenir le fonctionnement du système durant la suite du temps expiratoire du patient. A partir de ces considérations, le débit expiratoire minimum tel que la résistance de la troisième ouverture et la résistance de la valve expiratoire soit inférieure à la résistance de la deuxième ouverture peut être fixé à partir de 3L/min afin d'assurer l'ouverture de la valve dès le début de l'expiration du patient et maintenir le fonctionnement du système durant plus de 90% du temps expiratoire du patient. Le débit expiratoire maximal d'un patient standard respirant calmement correspond à 30L/min (pour un temps expiratoire de 2sec et un volume courant de 500mL) mais peut atteindre jusqu'à 600L/min lors d'une expiration forcée. Ainsi, dans le cas d'une inhalation calme, la résistance de la deuxième ouverture est avantageusement au minimum 10 fois supérieure à la résistance de la valve et à la résistance de la troisième ouverture pour un débit expiratoire de 30L/min. La deuxième ouverture peut être composée d'un orifice ou de plusieurs orifices.

Avantageusement, le générateur d'aérosol est placé sur la partie supérieure haute de la chambre afin de limiter la perte d'aérosol dans la chambre due au phénomène de sédimentation. En effet l'introduction de l'aérosol par le générateur d'aérosol dans la partie latérale d'une chambre d'inhalation accroît les pertes d'aérosol dans le dispositif (WO/0134232A1). Les particules introduites de manière latérale dans la partie centrale ou basse de la zone de stockage sédimenteront plus vite que les particules introduites dans la partie haute de la zone de stockage. La première ouverture destinée à être connectée au patient est également avantageusement placée sur la partie haute de la chambre, préférentiellement près du générateur d'aérosol afin d'assurer la vidange totale de la chambre après chaque inspiration. En effet, la vidange non totale du système induirait l'augmentation successive de la concentration de l'aérosol dans la chambre et aurait pour effet une augmentation du dépôt sur la surface de la zone de stockage par diffusion. La troisième ouverture comprenant la valve est préférentiellement placée proche de la première ouverture afin de limiter l'espace mort du système qui aurait pour conséquence l'asphyxie du patient par « rebreathing » ainsi que l'évacuation non totale de l'aérosol hors du système. Le volume maximum compris entre ces deux ouvertures peut être définie comme le volume mort anatomique standard de 200mL pour un adulte. La troisième ouverture comprenant la valve est donc avantageusement placée sur l'interface patient du type embout buccal, masque facial ou embout nasal mais peut également être dissociée de l'interface patient.

La deuxième ouverture destinée à assurer la pénétration de l'air dans la chambre d'inhalation lors de la phase inspiratoire est située de sorte qu'elle permette la ventilation complète de la zone de stockage afin de transporter la totalité de l'aérosol stocké dans la zone de stockage vers le patient.

La forme de la zone de stockage joue un rôle dans le dépôt par impaction des particules sur les parois internes lors de leurs transports. Une zone de stockage de forme cylindrique profilée dans un axe vertical, par exemple, a pour effet de diminuer ce phénomène. Cette forme cylindrique de la zone de stockage est particulièrement adaptée à la diffusion d'aérosol et limite le dépôt par impaction. L'utilisation de la forme cylindrique permet d'assurer un bon transport de l'aérosol hors de la zone de stockage et de limiter les zones de re-circulation de l'air, c'est à dire les zones de mauvaise ventilation dans la zone de stockage. Par ailleurs, afin d'assurer l'utilisation avantageuse de la forme cylindrique en association avec la deuxième ouverture, un deuxième cylindre englobant le premier cylindre peut être utilisé. Ce deuxième cylindre d'un diamètre plus large que le premier cylindre est muni uniquement de la deuxième ouverture en son sommet afin d'assurer la ventilation totale de l'intérieur de la chambre d'inhalation et de limiter la fuite de particules hors du système par cette deuxième ouverture lorsque le patient réalise des pauses respiratoires. La colonne d'air située dans l'espace compris entre le premier cylindre et le deuxième cylindre agit avec une certaine surpression limitant le déplacement des particules du premier cylindre vers le deuxième cylindre.

Ainsi le dispositif selon l'invention est particulièrement avantageux car la combinaison de ces caractéristiques permet de limiter la perte d'aérosol dans l'air ambiant par son stockage durant la phase expiratoire et de limiter les pertes par dépôt sur les parois. Cette zone de stockage combinée avec la position relative des ouvertures d'entrée d'air et de sortie d'aérosol permet donc d'augmenter la quantité d'aérosol stocké entre chaque inhalation et de limiter son dépôt sur les parois. Elle permet également de recevoir la génération d'un aérosol avec une vitesse relativement élevée sans perte sur les parois. Le volume dans lequel est projeté l'aérosol est compris entre 40mL et 500mL. Il est suffisamment grand pour que les particules générées avec une vitesse élevée aient le temps de ralentir par l'action du frottement de l'air, limitant ainsi leur dépôt par choc sur les parois du moyen complémentaire. Cette zone de stockage permet également de concentrer l'aérosol durant la phase expiratoire du patient, augmentant ainsi la quantité de principe actif qu'inhale le sujet à chaque inspiration et augmentant ainsi le débit du système. De plus, l'utilisation d'ouvertures avec des résistances différentes permet d'assurer une forte résistance à l'inspiration et une faible résistance à l'expiration du patient, ayant pour conséquence la réduction du débit inspiratoire, la diminution du dépôt oropharyngé et l'augmentation du dépôt pulmonaire.

L'invention permet donc d'augmenter non seulement le rendement du système de génération d'aérosol mais aussi l'efficacité de dépôt d'aérosol dans les poumons du patient par la résistance à l'écoulement de l'air durant l'inspiration du patient.

Ces caractéristiques et d'autres encore ressortiront bien de la suite de la description.
La Figure 1 illustre de manière schématique le principe de base de l'invention du dispositif en vue de profil,
La Figure 2 est une représentation schématique du dispositif en vue de profil, en cours de fonctionnement, comportant une interface du type embout buccal, au cours d'une phase d'expiration, le dispositif étant constitué de deux cylindres définissant la zone de stockage des particules,
La Figure 3 est une représentation schématique du dispositif en vue de profil, en cours de fonctionnement, comportant une interface du type embout buccal, au cours d'une phase d'inspiration, le dispositif étant constitué de deux cylindres définissant la zone de stockage des particules.
La Figure 4 est une représentation schématique du dispositif en vue en trois dimensions, comportant une interface du type embout buccal orthogonal, au cours d'une phase d'expiration, le dispositif étant constitué de deux cylindres définissant la zone de stockage des particules,
La Figure 5 est une représentation schématique du dispositif en vue en trois dimensions, comportant une interface du type embout buccal orthogonal, au cours d'une phase d'inspiration, le dispositif étant constitué de deux cylindres définissant la zone de stockage des particules,

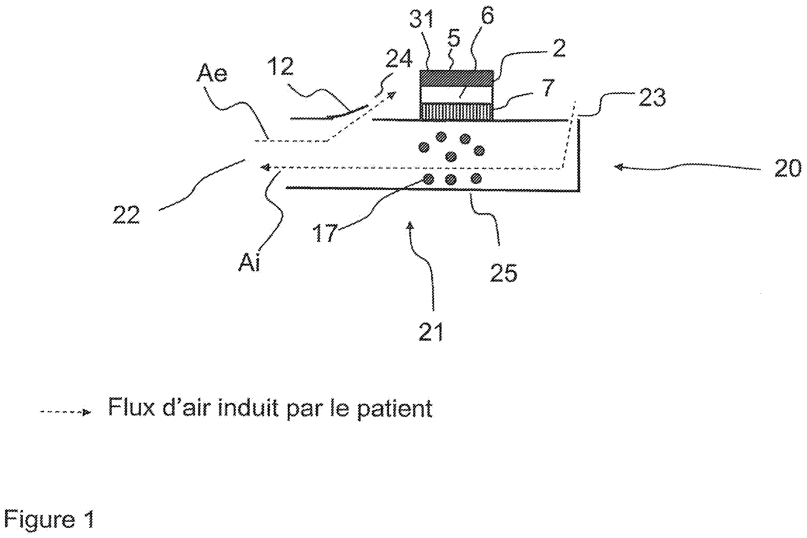

Selon la Figure 1, est illustré le principe de base de l'invention en vue de profil. Le dispositif (20) comprend un générateur d'aérosol (2) assurant la génération des particules (17) dans une chambre d'inhalation (21) comprenant 3 ouvertures, une première ouverture (22) destinée à être connectée au patient, une deuxième ouverture (23) permettant le passage de l'air extérieur dans la chambre d'inhalation (21), une troisième ouverture (24) munie d'une valve expiratoire unidirectionnelle (12). Le générateur d'aérosol (2) est un générateur à tamis fixe comprenant un réservoir (5) dans lequel le médicament (6) est introduit et un tamis (7) associé à un piézo électrique (31) dont la fréquence de vibration est comprise entre 50 KHz et 500 KHz et un tamis (7) dissocié du piézo électrique permettant de transformer le médicament liquide (6) en particules de médicaments (17) dans la zone de stockage (25) de la chambre d'inhalation (21).

Avantageusement la valve expiratoire (12) est composée d'une matière à mémoire de forme assurant sa fermeture à pression ambiante et permettant son ouverture pour une pression supérieure à 3cm d'H20 en association avec le dispositif (20). Par exemple, la valve expiratoire (12) a une résistance inférieure à 0.03 cm H20 min/L pour un débit expiratoire de 30L/min et est fabriquée en silicone ou élastomère. La valve peut également être réalisée à partir d'une matière solide non déformable et connectée à l'interface patient par l'intermédiaire d'une charnière à axe de rotation. Celle-ci se refermant par gravité et s'ouvrant à l'aide d'une légère surpression. Dans ce cas, la résistance de la valve expiratoire (12) est encore plus faible et a une résistance inférieure à 0.001 cmH20 min/L pour un débit expiratoire de 30L/min. La troisième ouverture (24) possède également une faible résistance. Compte tenu des débits expiratoires standards lors d'une séance d'inhalation, la troisième ouverture (24) a un diamètre préférentiel minimum de 5 mm. La deuxième ouverture (23) permettant le passage de l'air extérieur dans la chambre (21) a une résistance supérieure à la résistance de la troisième ouverture (24) associée à la valve (12).

Avantageusement, la résistance de la seconde ouverture (23) est 10 fois plus importante que la résistance de la troisième ouverture (24) associée à la valve (12) pour un débit expiratoire de 30L/min. Cette deuxième ouverture (23) peut être composée d'un orifice ou de plusieurs orifices. Le diamètre de l'orifice est préférentiellement inférieur à 20mm. Par ailleurs, afin de limiter les risques d'obstructions de ces orifices dues à leurs faibles tailles et assurer leur bon nettoyage après chaque séance d'inhalation, le diamètre de l'ouverture d'un orifice est au minimum de 1mm. Par exemple, la résistance équivalente de 6 orifices d'un diamètre de 2 mm est de 6 cm H20 min/L pour un débit de 30L/min. Ainsi durant la phase expiratoire du patient, la résistance de la valve (12) et de la troisième ouverture (24) étant moins importante que la résistance de la deuxième ouverture (23), l'air expiré (Ae) par le patient à travers la première ouverture (22) est expulsé préférentiellement par la troisième ouverture (24) sans traverser la zone de stockage (25). Les particules (17) produites par le générateur d'aérosol (2) sont stockées dans la zone de stockage (25) durant la phase expiratoire du patient. Durant la phase inspiratoire du patient, la valve expiratoire (12) ferme la troisième ouverture (24) et l'air inspiré (Ai) par le patient passe par la deuxième ouverture (23) transportant avec lui les particules (17) produites et stockées dans la zone de stockage (25) vers le patient à travers la première ouverture (22).

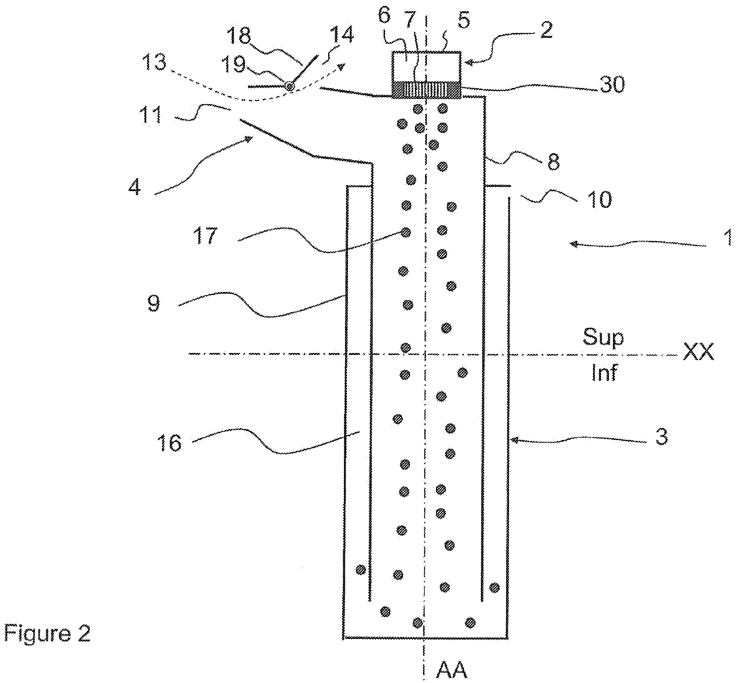

Selon la Figure 2, est illustré le dispositif (1) selon un mode de réalisation optimisée de l'invention. Le dispositif comprend un générateur d'aérosol (2), une zone de stockage (3) et une interface (4) de type embout buccal. Le générateur d'aérosol est un générateur à tamis vibrant connu de l'art antérieur comprenant un réservoir (5) dans lequel le médicament (6) est introduit et un tamis (7) mis en vibration par un piézo électrique (30) à une fréquence comprise entre 50 KHz et 500 KHz permettant de transformer le médicament liquide en particules de médicaments (17).

Avantageusement, le générateur d'aérosol est placé sur la partie supérieure haute (Sup) de la zone de stockage (3) afin de limiter la perte d'aérosol dans la zone de stockage due au phénomène de sédimentation. Cette partie supérieure (Sup) est définie par rapport à l'axe transversal médian (XX) établi sur la hauteur du dispositif. La zone de stockage (3) est préférentiellement composée de deux cylindres inclus l'un dans l'autre et profilés et disposés selon un axe vertical (AA). Le premier cylindre (8) est ouvert de part et d'autre. Le deuxième cylindre (9) contenant le premier (8) comporte uniquement une ouverture (10) en sa partie supérieure haute. La forme de cette zone de stockage (3) est particulièrement adaptée à la diffusion d'aérosol et limite le dépôt par impaction. Compte tenu du débit du générateur d'aérosol et du débit inspiratoire du patient, le premier cylindre (8) a préférentiellement un diamètre compris entre 20mm et 100mm. Avantageusement le diamètre du deuxième cylindre (9) est inférieur à trois fois le diamètre du premier cylindre (8) de telle sorte que la distance entre les parois de chacun des deux cylindres (8 et 9) soit plus faible que le diamètre du premier cylindre (8) afin de limiter le volume d'aérosol stocké entre les deux parois (16). En effet l'aérosol qui est stocké entre les deux parois (16) aura un fort risque d'impaction dans la partie basse du deuxième cylindre (9) lors de son transport en phase inspiratoire. De plus afin de limiter la sédimentation de l'aérosol dans le bas du dispositif (1), la zone de stockage a une hauteur minimum de 40mm et l'espace entre le bas du premier cylindre (8) et la surface basse du deuxième cylindre (9) est au minimum de 1mm.

L'interface (4) de type embout buccal est placée sur la partie supérieure latérale de la zone de stockage (3) préférentiellement près du générateur d'aérosol afin d'assurer la vidange totale de la chambre après chaque inspiration.

L'interface (4) comprend une ouverture (14) et une valve expiratoire non déformable (18) munie d'une charnière à axe de rotation (19) ouvrant l'ouverture (14) lors de la phase expiratoire et fermant l'ouverture (14) lors de la phase inspiratoire et lors des phases de pauses respiratoires.

La Figure 2 représente le fonctionnement dans cette configuration optimisée de l'invention lors de la phase expiratoire du patient. Le médicament liquide (6) est introduit dans le réservoir (5) du générateur d'aérosol et les particules (17) sont produites à travers le tamis vibrant (7) selon l'axe (AA) du cylindre (8). Due au poids et aux vitesses initiales des particules, une force dirigée vers le bas du dispositif est appliquée sur les particules (17). A l'opposé, une force de pression provenant de la colonne d'air comprise entre les deux cylindres (8 et 9) est appliquée vers le bas de l'espace (16). Cette force opposée à la première a pour effet de limiter le déplacement des particules vers l'espace située entre les deux cylindres (16) et également vers l'ouverture (10) en contact avec l'air extérieur. Le patient expire par la bouche dans le dispositif par l'ouverture (11) en extrémité de l'interface (4). L'air expiré (13) par le patient crée une légère surpression dans le dispositif. La résistance de l'ouverture (14) et de la valve (18) étant moins élevée que la résistance de l'ouverture (10), la valve (18) se soulève et permet d'ouvrir l'ouverture (14). L'air expiré (13) par le patient est expulsé hors du dispositif par l'ouverture (14). L'air expiré (13) ne traverse pas la zone de stockage (3) et les particules produites (17) sont stockées dans la zone de stockage (3) pour l'inhalation suivante.

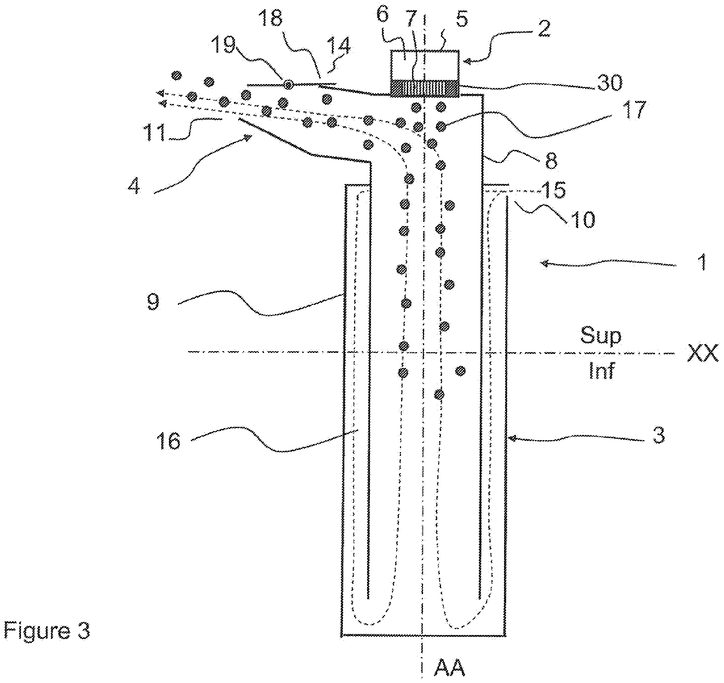

La Figure 3 représente le fonctionnement du dispositif dans une mise en oeuvre optimisée de l'invention lors de la phase inspiratoire du patient. Le dispositif (1) représenté dans la Figure 3 est identique au dispositif (1) représenté dans la Figure 2. Les particules (17) sont générées dans la zone de stockage (3) par le générateur d'aérosol (2). Le patient est connecté par l'ouverture (11) de l'interface (4) et lors de la phase inspiratoire, la valve (18) ferme l'ouverture (14). L'air inspiré (15) par le patient connecté à l'ouverture (11) de l'interface (4) pénètre dans le dispositif (1) par l'ouverture (10) en créant une résistance à l'inspiration de façon à limiter le débit inspiratoire du patient à une faible valeur. L'air (15) passe dans le deuxième cylindre (9) puis dans le premier cylindre (8) pour être inspiré par le patient à travers l'ouverture (11). En traversant le dispositif (1), l'air (15) transporte avec lui les particules (17) générées par le générateur d'aérosol (2) et stockées dans la zone de stockage (3) durant la phase expiratoire (Figure 2) précédent cette phase inspiratoire (Figure 3).

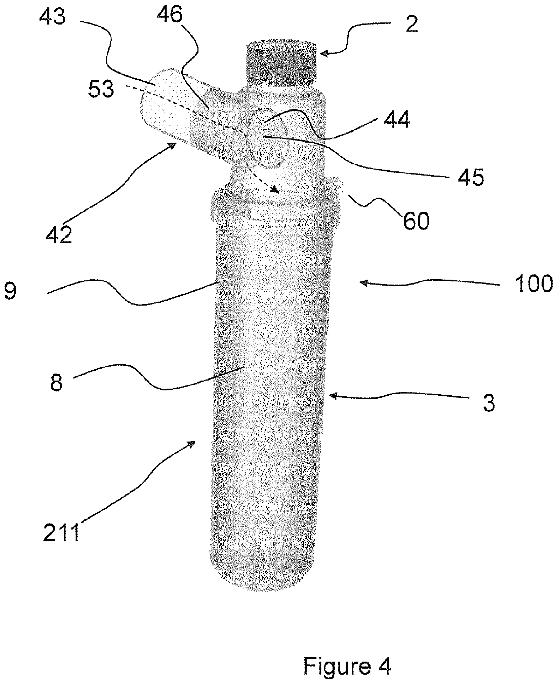

Selon la Figure 4, est illustré le dispositif (100) selon un mode de réalisation de l'invention avec un embout buccal de type orthogonal (42). Le dispositif (100) comprend un générateur d'aérosol (2) assurant la génération des particules (17) dans une chambre d'inhalation (211). Cette chambre de d'inhalation (211) est composée d'une zone de stockage (3) et d'une interface de type embout buccal (42). Le générateur d'aérosol est un générateur à tamis vibrant (2).

L'interface (42) de type embout buccal orthogonal est muni d'une ouverture (43) destiné à être connectée au patient, d'une ouverture (44) munie d'une valve expiratoire unidirectionnelle de faible résistance (45) permettant l'expiration du patient par l'ouverture (44) et d'une section de passage (46) permettant le passage de l'aérosol depuis la zone de stockage (3) vers l'embout buccal (42). L'axe reliant les sections de passages des ouvertures (43) et (44) est perpendiculaire à l'axe de la section de passage (46). L'air expiré (53) par le patient est dirigé selon un axe rectiligne en direction de l'ouverture (44) et crée une légère surpression dans le dispositif. La résistance de l'ouverture (44) et de la valve (45) étant moins élevée que la résistance de la seconde ouverture (60), la valve (45) se soulève et permet d'ouvrir l'ouverture (44). L'air expiré (53) par le patient est dirigé de manière rectiligne vers l'ouverture (44) et est expulsé hors du dispositif (1) par l'ouverture (44). L'air expiré (53) ne traverse pas la zone de stockage (3) et les particules produites sont stockées dans la zone de stockage (3) pour l'inhalation suivante.

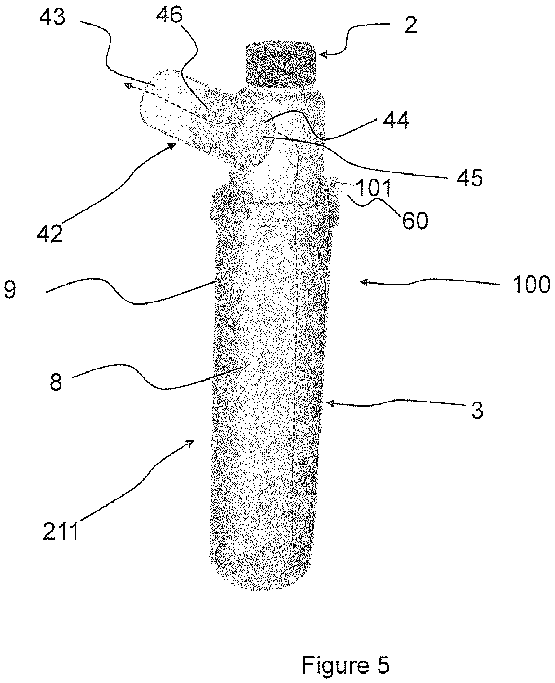

La Figure 5 représente le fonctionnement du dispositif dans une mise en oeuvre de l'invention comportant une interface embout buccal orthogonal, lors de la phase inspiratoire du patient. Le dispositif (100) représenté dans la Figure 5 est identique au dispositif (100) représenté dans la Figure 4. Les particules sont générées dans la zone de stockage (3) par le générateur d'aérosol à tamis vibrant (2). Le patient est connecté à l'ouverture (43) de l'interface (42). Lors de la phase inspiratoire, la valve (45) ferme l'ouverture (44). L'air inspiré (101) par le patient connecté à l'ouverture (43) de l'interface (42) pénètre dans le dispositif (100) par la seconde ouverture (60) en créant une résistance à l'inspiration de façon à limiter le débit inspiratoire du patient à une faible valeur. L'air (101) passe dans le deuxième cylindre (9) puis dans le premier cylindre (8) pour être inspiré par le patient à travers la section de passage (46) puis l'ouverture (43). En traversant le dispositif (100), l'air (101) transporte avec lui les particules générées par le générateur d'aérosol (2) et stockées dans la zone de stockage (3) durant la phase expiratoire (Figure 4) précédent cette phase inspiratoire (Figure 5).

Dans les configurations précitées, la disposition des ouvertures et de la valve peuvent varier en position, les figures ayant été décrites et citées à titre d'exemple. Par exemple, l'interface (4) pourrait être placée sur la partie supérieure horizontale de la zone de stockage. Par exemple, l'interface peut être une pièce en forme de T telle que la valve (18) soit située sur l'ouverture située en face de l'ouverture (11) destinée à être connectée au patient. L'autre ouverture de la pièce en T étant connectée à la zone de stockage et étant disposée dans un axe perpendiculaire à l'axe reliant les deux autres ouvertures. De même, la valve (18) peut être dissociée de l'interface (4). Par exemple, un masque facial peut être connecté à la pièce en T précédemment définie. Par ailleurs, il est à noter, qu'aucune valve n'est placée sur le trajet de l'aérosol lors de son administration au patient, permettant ainsi de supprimer les pertes de particules de médicament par dépôt sur valve durant la phase inspiratoire du patient. La nébulisation par tamis est actuellement réalisée à l'aide d'un piézo électrique, mais l'utilisation d'une autre technologie pour mettre en mouvement le liquide ou le tamis pourrait être appliquée à ce type de nébuliseur à tamis. A titre d'exemple, nous citons l'utilisation d'une source de pression dans le réservoir utilisé dans l'aérosol doseur Respimat® de Boehringer Ingelheim.

Les avantages ressortent bien de l'invention qui répond parfaitement aux objectifs de réduction des pertes d'aérosols.

## Revendications

1. Dispositif de nébulisation (20;1;100) comprenant un générateur d'aérosol (2) à tamis (7) associé à une chambre d'inhalation (21;211), et un réservoir (5) destiné à recevoir un médicament (6) sous forme liquide, le tamis (7) connecté au réservoir (5) et en contact avec le médicament (6) et permettant le passage du médicament (6) puis la génération de particules (17) de médicaments de l'autre côté du tamis (7) dans la chambre d'inhalation (21;211), **caractérisé en ce que** la chambre d'inhalation (21;211) est constituée avec trois ouvertures et une unique valve, ladite valve étant une valve expiratoire unidirectionnelle (12;18;45), une première ouverture (22;11;43) étant destinée à être connectée au patient et à assurer le transport et l'administration des particules (17) depuis le dispositif vers le patient, la dite première ouverture (22;11;43) étant placée en aval du générateur d'aérosol (2), une seconde ouverture (23;10;60) étant placée en amont du générateur d'aérosol (2) du dispositif et permettant le passage de l'air depuis l'extérieur de la chambre vers l'intérieur de la chambre d'inhalation (21;211), et permettant la ventilation complète de l'intérieur de la chambre d'inhalation (21;211), une troisième ouverture (24;14;44) étant placée en aval du générateur d'aérosol (2) et en amont de la dite première ouverture (22;11;43), et étant munie de la valve expiratoire unidirectionnelle (12;18;45) permettant l'expiration de l'air du patient par cette troisième ouverture (24;14;44), la dite troisième ouverture (24;14;44) étant fermée à l'aide de la valve expiratoire unidirectionnelle (12;18;45) lors des phases inspiratoires et ouverte durant les phases expiratoires, et **en ce que** la résistance de la dite troisième ouverture (24;14;44) associée à celle de la valve expiratoire unidirectionnelle (12;18;45) est inférieure à la résistance de la dite seconde ouverture (23;10;60) afin de limiter l'échappement des particules (17) par la dite seconde ouverture (23;10;60) qui ont été produites dans la chambre d'inhalation (21;211), **et en ce que** la seconde ouverture (23;10;60) a pour fonction de créer une résistance à l'air inspiré par le patient afin de limiter le débit de l'air inspiré par le patient.

2. Dispositif (20;1;100) selon la revendication 1, **caractérisé en ce que** la valve expiratoire (12;18;45) est composée d'une matière à mémoire de forme assurant sa fermeture à pression ambiante.

3. Dispositif (20;1;100) selon l'une quelconque des revendications 1 et 2, **caractérisé en ce que** la valve expiratoire (12;18;45) est réalisée à partir d'une matière solide non déformable.

4. Dispositif (20;1;100) selon la revendication 1, **caractérisé en ce que** le volume compris entre la première ouverture (22;11;43) et la troisième ouverture (24;14;44) est inférieur à 200mL.

5. Dispositif (20;1;100) selon la revendication 1, **caractérisé en ce que** la résistance de la troisième ouverture (24;14;44) et de la valve expiratoire (12;18;45) est inférieure à 0.07 cm H20 min/L.

6. Dispositif (20;1;100) selon la revendication 1, **caractérisé en ce que** la résistance de la troisième ouverture (24;14;44) associée à celle de la valve expiratoire unidirectionnelle (12;18;45) est inférieure à la résistance de la deuxième ouverture (23;10;60) pour des débits expiratoires supérieurs à 3 L/min.

7. Dispositif (20;1;100) selon la revendication 1, **caractérisé en ce que** la résistance de la troisième ouverture (24;14;44) munie de la valve expiratoire unidirectionnelle (12;18;45) a une résistance au moins 10 fois moins importante que la résistance de la deuxième ouverture (23;10;60) pour un débit expiratoire supérieur à 30L/min.

8. Dispositif (20;1;100) la revendication 1, **caractérisé en ce que** la chambre d'inhalation (21;211) un volume compris entre 40mL et 500mL

9. Dispositif (1) selon l'une quelconque des revendications 1 à 8, **caractérisé en ce que** le générateur d'aérosol (2) et la première ouverture (11) sont tout deux disposés sur la partie supérieure du dispositif, **et en ce que** la chambre d'inhalation comporte une zone de stockage (3) constituée de deux cylindres (8, 9) inclus l'un dans l'autre et disposés selon un axe vertical, le dit premier cylindre (8) étant ouvert de part et d'autre, le dit second cylindre (9) contenant le premier cylindre (8) et comportant uniquement une ouverture (10) dans sa partie supérieure haute, la dite ouverture formant la seconde ouverture (10) de la chambre d'inhalation, **et en ce que** le dit premier cylindre (8) a un diamètre inférieur au deuxième cylindre (9), **et en ce que** le deuxième cylindre (9) comprend le premier cylindre (8), **et en ce que** la troisième ouverture (14) est située dans la partie supérieure du dispositif.

10. Dispositif (1) selon la revendication 9, **caractérisé en ce qu'il** comprend une interface (4) placée sur la partie supérieure de la zone de stockage (3) près du générateur d'aérosol (2), la dite interface (4) comprenant la dite troisième ouverture (14) et la valve expiratoire unidirectionnelle (18), la dite valve expiratoire unidirectionnelle (18) étant une valve non déformable munie d'une charnière à axe de rotation (19) ouvrant l'ouverture (14) lors de la phase expiratoire et fermant l'ouverture (14) lors de la phase inspiratoire et lors des phases de pauses respiratoires.

11. Dispositif (1) selon la revendication 10, **caractérisé en ce que** l'interface (4) est un embout buccal.

12. Dispositif (1) selon la revendication 9, **caractérisé en ce que** le premier cylindre (8) a un diamètre compris entre 20mm et 100mm et une hauteur minimum de 40mm, **et en ce que** le deuxième cylindre (9) a un diamètre inférieur à 3 fois le diamètre du premier cylindre (8), **et en ce que** la deuxième ouverture (10) a un diamètre compris entre 1mm et 20mm.

13. Dispositif (20;1;100) selon la revendication 1, **caractérisé en ce que** la deuxième ouverture (23;10;60) a un diamètre compris entre 1mm et 20mm.

14. Dispositif (20;1;100) selon la revendication 1, **caractérisé en ce que** la troisième ouverture (24;14;44) munie de la valve expiratoire unidirectionnelle (12;18;45) a une résistance inférieure à 0.03 cm H20 min/L pour un débit d'air expiré de 30L/min.

15. Dispositif (20;1;100) selon la revendication 1, **caractérisé en ce que** la deuxième ouverture (23;10;60) est située en amont de la première ouverture (22;11;43), la troisième ouverture (24;14;44) est située en aval de la deuxième ouverture (23;10;60) et en amont de la première ouverture (22;11;43), **et en ce que** le tamis (7) est placé en aval de la deuxième ouverture (23;10;60) et en amont de la troisième ouverture (24;14;44).

16. Dispositif (20) selon la revendication 1, **caractérisé en ce qu'un** piézo électrique (31) est associé au réservoir (5) et non connecté au tamis (7) afin de mettre en mouvement le liquide de médicament pour réaliser une génération de particules à travers le tamis (7).

17. Dispositif (1) selon la revendication 1, **caractérisé en ce qu'un** piézo électrique (30) est connecté au tamis (7) afin de mettre en mouvement le tamis (7) pour réaliser une génération de particules à travers le tamis (7).

18. Dispositif (20;1) selon l'une des revendications 16 et 17, **caractérisé en ce que** la fréquence de vibration du piézo électrique (31;30) est comprise entre 50KHz et 500KHz.

## Patentansprüche

1. Vernebelungsvorrichtung (20;1;100) bestehend aus einer Aerosolpackung (2) mit Sieb (7) verbunden mit einer Inhalationskammer (21;211) und einem Reservoir (5) zur Aufnahme eines Medikaments (6) in flüssiger Form, wobei das Sieb (7) am Reservoir (5) angeschlossen und mit dem Medikament (6) in Kontakt ist und den Durchlass des Medikaments (6) und anschließend die Erzeugung von Medikament-Partikeln (17) auf der anderen Seite des Siebes (7) in der Inhalationskammer (21;211) ermöglicht, **dadurch gekennzeichnet, dass** die Inhalationskammer (21;211) mit drei Öffnungen und einem einzigen Ventil versehen ist, wobei es sich bei dem Ventil um ein Einweg-Ausatemventil (12;18;45) handelt, eine erste Öffnung (22;11;43) zum Anschluss an den Patienten und zum Transport und zur Verabreichung der Partikel (17) von der Vorrichtung hin zum Patienten bestimmt ist, wobei diese erste Öffnung (22;11;43) stromabwärts der Aerosolpackung (2) angeordnet ist, eine zweite Öffnung (23;10;60) stromaufwärts der Aerosolpackung (2) der Vorrichtung angeordnet ist und den Durchlass der Luft von außerhalb der Kammer in das Innere der Inhalationskammer (21;211) ermöglicht und die komplette Luftzirkulation innerhalb der Inhalationskammer (21;211) zulässt, eine dritte Öffnung (24;14;44) stromabwärts der Aerosolpackung (2) und stromaufwärts der ersten Öffnung (22;11;43) angeordnet und mit dem Einweg-Ausatemventil (12;18;45) versehen ist und ein Ausatmen der Luft des Patienten über diese dritte Öffnung (24;14;44) zulässt, wobei diese dritte Öffnung (24;14;44) über das Einweg-Ausatemventil (12;18;45) bei den Inspirationsphasen geschlossen und bei den Exspirationsphasen geöffnet ist, **und dass** die Widerstandskraft der dritten Öffnung (24;14;44) zusammen mit der des Einweg-Ausatemventils (12;18;45) geringer als die Widerstandskraft der zweiten Öffnung (23;10;60) ist, um ein Entweichen der in der Inhalationskammer (21;211) erzeugten Partikel (17) durch die zweite Öffnung (23;10;60) einzuschränken **und dass** die zweite Öffnung (23;10;60) die Aufgabe hat, gegen die vom Patienten eingeatmete Luft einen Widerstand aufzubauen und dadurch den Durchsatz der vom Patienten eingeatmeten Luft zu begrenzen.

2. Vorrichtung (20;1;100) nach Anspruch 1, **dadurch gekennzeichnet, dass** das Ausatemventil (12;18;45) aus einem Formgedächtnismaterial besteht, der ein Verschließen desselben bei Umgebungsdruck sicherstellt.

3. Vorrichtung (20;1;100) nach Anspruch 1 oder 2, **dadurch gekennzeichnet, dass** das Ausatemventil (12;18;45) aus einem festen, nicht verformbaren Material gebildet ist.

4. Vorrichtung (20;1;100) nach Anspruch 1, **dadurch gekennzeichnet, dass** das Volumen zwischen der ersten Öffnung (22;11;43) und der dritten Öffnung (24;14;44) weniger als 200 ml beträgt.

5. Vorrichtung (20;1;100) nach Anspruch 1, **dadurch gekennzeichnet, dass** die Widerstandskraft der dritten Öffnung (24;14;44) und des Ausatemventils (12;18;45) weniger als 0,07 cm H20 min/l beträgt.

6. Vorrichtung (20;1;100) nach Anspruch 1, **dadurch gekennzeichnet, dass** die Widerstandskraft der dritten Öffnung (24;14;44) zusammen mit der des Einweg-Ausatemventils (12;18;45) bei Ausatmungsdurchsätzen von über 3 l/min geringer als die Widerstandskraft der zweiten Öffnung (23;10;60) ist.

7. Vorrichtung (20;1;100) nach Anspruch 1, **dadurch gekennzeichnet, dass** die Widerstandskraft der mit dem Einweg-Ausatemventil (12;18;45) versehenen dritten Öffnung (24;14;44) bei einem Ausatmungsdurchsatz von über 30 l/min. eine 10mal kleinere Widerstandskraft als die Widerstandskraft der zweiten Öffnung (23;10;60) aufweist.

8. Vorrichtung (20;1;100) nach Anspruch 1, **dadurch gekennzeichnet, dass** die Inhalationskammer (21;211) ein Volumen zwischen 40 ml und 500 ml aufweist.

9. Vorrichtung (1) nach einem der Ansprüche 1 bis 8, **dadurch gekennzeichnet, dass** die Aerosolpackung (2) und die erste Öffnung (11) beide im oberen Bereich der Vorrichtung angeordnet sind, **und dass** die Inhalationskammer einen Vorratsbereich (3) umfasst, der aus zwei, entlang einer Vertikalachse angeordneten, einander aufnehmenden zylindrischen Behältern (8) und (9) gebildet wird, wobei der erste zylindrische Behälter (8) zu beiden Seiten hin offen ist und der zweite zylindrische Behälter (9), der den ersten zylindrischen Behälter aufnimmt, nur in seinem oberen Bereich eine Öffnung (10) aufweist, wobei diese Öffnung die zweite Öffnung (10) der Inhalationskammer bildet, **und dass** der Durchmesser des ersten zylindrischen Behälters (8) kleiner als der Durchmesser des zweiten zylindrischen Behälters (9) ist, **und dass** der zweite zylindrische Behälter (9) den ersten zylindrischen Behälter (8) aufnimmt, **und dass** sich die dritte Öffnung (14) im oberen Bereich der Vorrichtung befindet.

10. Vorrichtung (1) nach Anspruch 9, **dadurch gekennzeichnet, dass** die Vorrichtung eine Schnittstelle (4) aufweist, die im oberen Teil des Vorratsbereichs (3) nahe der Aerosolpackung (2) platziert ist, wobei diese Schnittstelle (4) die dritte Öffnung (14) und das Einweg-Ausatemventil (18) umfasst, wobei es sich bei dem Einweg-Ausatemventil (18) um ein nicht verformbares Ventil handelt, das mit einem Scharnier mit Drehachse (19) versehen ist, welches die Öffnung (14) während der Exspirationsphase öffnet und die Öffnung (14) während der Inspirationsphase und während der Atempausenphasen schließt.

11. Vorrichtung (1) nach Anspruch 10, **dadurch gekennzeichnet, dass** es sich bei der Schnittstelle (4) um ein Mundstück handelt.

12. Vorrichtung (1) nach Anspruch 9, **dadurch gekennzeichnet, dass** der erste zylindrische Behälter (8) einen Durchmesser von 20 mm bis 100 mm und eine Höhe von mindestens 40 mm aufweist, **und dass** der zweite zylindrische Behälter (9) einen 3mal kleineren Durchmesser als der erste zylindrische Behälter (8) aufweist, **und dass** die zweite Öffnung (10) einen Durchmesser von 1 mm bis 20 mm aufweist.

13. Vorrichtung (20;1;100) nach Anspruch 1, **dadurch gekennzeichnet, dass** die zweite Öffnung (23;10;60) einen Durchmesser von 1 mm bis 20 mm aufweist.

14. Vorrichtung (20;1;100) nach Anspruch 1, **dadurch gekennzeichnet, dass** die dritte Öffnung (24;14;44), versehen mit dem Einweg-Ausatemventil (12;18;45), bei einem Ausatmungsdurchsatz von 30 l/min eine Widerstandskraft von unter 0,03 cm H20 min/l aufweist.

15. Vorrichtung (20;1;100) nach Anspruch 1, **dadurch gekennzeichnet, dass** die zweite Öffnung (23;10;60) stromaufwärts der ersten Öffnung (22;11;43), und die dritte Öffnung (24;14;44) stromabwärts der zweiten Öffnung (23;10;60) und stromaufwärts der ersten Öffnung (22;11;43) angeordnet ist, **und dass** das Sieb (7) stromabwärts der zweiten Öffnung (23;10;60) und stromaufwärts der dritten Öffnung (24;14;44) angeordnet ist.

16. Vorrichtung (20) nach Anspruch 1, **dadurch gekennzeichnet, dass** ein Piezoelektrikum (31) dem Reservoir (5) zugeordnet und nicht an das Sieb (7) angeschlossen ist, um die Medikamentenflüssigkeit zur Erzeugung von Partikeln durch das Sieb (7) hindurch in Bewegung zu setzen.

17. Vorrichtung (1) nach Anspruch 1, **dadurch gekennzeichnet, dass** ein Piezoelektrikum (30) an Sieb (7) angeschlossen ist, um das Sieb zur Erzeugung von Partikeln durch das Sieb (7) hindurch in Bewegung zu setzen.

18. Vorrichtung (20;1) nach Anspruch 16 oder 17, **dadurch gekennzeichnet, dass** die Schwingungsfrequenz des Piezoelektrikums zwischen 50 KHz und 500 KHz liegt.

## Claims

1. A nebulizer device (20; 1; 100) comprising an aerosol generator (2) having a screen (7) associated with an inhalation chamber (21, 211), and a reservoir (5) designed to receive medication (6) in liquid form, the screen (7) being connected to the reservoir (5) and in contact with the medication (6), and enabling the medication (6) to pass through it, and then particles (17) of medication to be generated on the other side of the screen (7) in the inhalation chamber (21; 211), said nebulizer device being **characterized in that** the inhalation chamber (21; 211) is provided with three openings and a single valve, said valve being a one-way exhalation valve (12; 18; 45), a first opening (22; 11; 43) being designed to be connected to the patient and to convey and administer the particles (17) from the device to the patient, said first opening (22; 11; 43) being placed downstream from the aerosol generator (2), a second opening (23; 10; 60) being placed upstream from the aerosol generator (2) of the device, and enabling air to pass through it from the outside of the inhalation chamber (21; 211) to the inside of the inhalation chamber (21; 211), and enabling the inside of the inhalation chamber (21; 211) to be fully ventilated, a third opening (24; 14; 44) being placed downstream from the aerosol generator (2) and upstream from said first opening (22; 11; 43) and being provided with the one-way exhalation valve (12; 18; 45) enabling air to be exhaled from the patient via said third opening (24; 14; 44), said third opening (24; 14; 44) being closed by means of the one-way exhalation valve (12; 18; 45) during the inhalation phases and open during the exhalation phases, and **in that** the resistance of said third opening (24; 14; 44) associated with the resistance of said one-way exhalation valve (12; 18; 45) is less than the resistance of said second opening (23; 10; 60) in order to limit the discharge via said second opening (23; 10; 60) of particles (17) that have been generated in the inhalation chamber (21; 211), and **in that** the function of the second opening (23; 10; 60) is to create resistance to the air inhaled by the patient in order to limit the flow rate of air inhaled by the patient.

2. A device (20; 1; 100) according to claim 1, **characterized in that** the exhalation valve (12; 18; 45) is made of a shape-memory material that causes it to close at ambient pressure.

3. A device (20; 1; 100) according to claim 1 or claim 2, **characterized in that** the exhalation valve (12; 18; 45) is made of a non-deformable solid material.

4. A device (20; 1; 100) according to claim 1, **characterized in that** the volume lying between the first opening (22; 11; 43) and the third opening (24; 14; 44) is less than 200 milliliters (mL).

5. A device (20; 1; 100) according to claim 1, **characterized in that** the resistance of the third opening (24; 14; 44) and of the exhalation valve (12; 18; 45) is less than 0.07 centimeters of water per minute per liter (cm H₂O min/L).

6. A device (20; 1; 100) according to claim 1, **characterized in that** the resistance of the third opening (24; 14; 44) associated with the resistance of the one-way exhalation valve (12; 18; 45) is less than the resistance of the second opening (23; 10; 60) for exhalation flow rates greater than 3 liters per minute (L/min).

7. A device (20; 1; 100) according to claim 1, **characterized in that** the resistance of the third opening (24; 14; 44) provided with the one-way exhalation valve (12; 18; 45) is at least 10 times lower than the resistance of the second opening (23; 10; 60) for an exhalation flow rate greater than 30 L/min.

8. A device (20; 1; 100) according to claim 1, **characterized in that** the inhalation chamber (21; 211) has a volume lying in the range 40 mL to 500 mL.

9. A device (1) according to any one of claims 1 to 8, **characterized in that** the aerosol generator (2) and the first opening (11) are both disposed on the top portion of the device, and **in that** the inhalation chamber has a storage zone (3) made up of two cylinders (8, 9) nested one in the other, said second cylinder (9) containing the first cylinder (8) and being provided with only one opening (10) in its top portion, which opening forms the second opening (10) of the inhalation chamber, and **in that** said first cylinder (8) has a diameter less than the diameter of the second cylinder (9), and **in that** the second cylinder (9) includes the first cylinder (8), and **in that** the third opening (14) is situated in the top portion of the device.

10. A device (1) according to claim 9, **characterized in that** it further comprises an interface (4) placed on the top portion of the storage zone (3) close to the aerosol generator (2), said interface (4) being provided with said third opening (14) and with the one-way exhalation valve (18), said one-way exhalation valve (18) being a non-deformable valve provided with a pivot-pin hinge (19) opening the opening (14) during the exhalation phase and closing the opening (14) during the inhalation phase, and during breathing pause phases.

11. A device (1) according to claim 10, **characterized in that** the interface (4) is a mouthpiece.

12. A device (1) according to claim 9, **characterized in that** the first cylinder (8) has a diameter lying in the range 20 millimeters (mm) to 100 mm, and a minimum height of 40 mm, and **in that** the second cylinder (9) has a diameter less than 3 times the diameter of the first cylinder (8), and **in that** the second opening (10) has a diameter lying in the range 1 mm to 20 mm.

13. A device (20; 1; 100) according to claim 1, **characterized in that** the second opening (23; 10; 60) has a diameter lying in the range 1 mm to 20 mm.

14. A device (20; 1; 100) according to claim 1, **characterized in that** the third opening (24; 14; 44) provided with the one-way exhalation valve (12; 18; 45) has a resistance less than 0.03 cm H₂O min/L for a flow rate of exhaled air of 30 L/min.

15. A device (20; 1; 100) according to claim 1, **characterized in that** the second opening (23; 10; 60) is situated upstream from the first opening (22; 11; 43), the third opening (24; 14; 44) is situated downstream from the second opening (23; 10; 60) and upstream from the first opening (22; 11; 43), and **in that** the screen (7) is placed downstream from the second opening (23; 10; 60) and upstream from the third opening (24; 14; 44).

16. A device (20) according to claim 1, **characterized in that** a piezoelectric (31) is associated with the reservoir (5) and is not connected to the screen (7) in order to cause the medication liquid to move so as to cause particles to be generated through the screen (7).

17. A device (1) according to claim 1, **characterized in that** a piezoelectric (30) is connected to the screen (7) in order to cause the screen to move so as to cause particles to be generated through the screen (7).

18. A device (20; 1) according to claim 16 or claim 17, **characterized in that** the frequency of vibration of the piezoelectric lies in the range 50 kilohertz (kHz) to 500 kHz.
